Europäisches Patentamt

European Patent Office

Office européen des brevets

(19)

(11) Publication number: **0 279 519**
**A1**

# EUROPEAN PATENT APPLICATION

(21) Application number: 88300478.0

(22) Date of filing: **21.01.88**

(51) Int. Cl.⁴: **A61K 31/19 , A61K 47/00**

The title of the invention has been amended (Guidelines for Examination in the EPO, A-III, 7.3).

(30) Priority: **22.01.87 GB 8701392**

(43) Date of publication of application:
**24.08.88 Bulletin 88/34**

(84) Designated Contracting States:
**AT BE CH DE ES FR GB GR IT LI LU NL SE**

(71) Applicant: **THE BOOTS COMPANY PLC**
**1 Thane Road West**
**Nottingham NG2 3AA(GB)**

(72) Inventor: **Smith, Alan**
**20 May Avenue**
**Wollaton Nottingham Nottinghamshire(GB)**

(74) Representative: **Thacker, Michael Anthony et al**
**THE BOOTS COMPANY PLC Patents Section**
**R4 Pennyfoot Street**
**Nottingham NG2 3AA(GB)**

(54) **Pharmaceutical compositions containing salts of ibuprofen in a hydrophilic carrier.**

(57) The use of a pharmaceutically acceptable salt of ibuprofen in an ibuprofen formulation comprising a carrier which is a hydrophilic material selected from an alcohol or an ester thereof and a polyhydric alcohol or an ester thereof to stabilise the formulation.
Preferably the sodium salt is employed.

EP 0 279 519 A1

# Therapeutic Agents

This invention relates to therapeutically effective formulations comprising a pharmaceutically acceptable salt of ibuprofen.

Ibuprofen, the chemical name of which is 2-(4-isobutylphenyl)propionic acid, is a well-known medicament with anti-inflammatory, antipyretic and analgesic activities. The uses of ibuprofen and its pharmaceutically acceptable salts include the treatment of pain and inflammation in musculoskeletal disorders such as rheumatic disease, and the treatment of pain in a variety of other disorders, for example headache, neuralgia and dysmenorrhoea.

Ibuprofen is generally formulated with pharmaceutically acceptable excipients to form a presentation suitable for administration to a patient. The pharmaceutically acceptable excipients may be selected from a wide range of excipients, chosen according to the desired form of presentation, i.e. whether a solid, semi-solid or liquid formulation is desired or whether a solid formulation is in the form of a tablet, a capsule or a suppository etc.. Some pharmaceutically acceptable excipients fall within a class of excipients which are hydrophilic materials, and in particular are alcohols or esters thereof, or polyhydric alcohols or esters thereof.

Surprisingly, it has now been found that when a pharmaceutically acceptable salt of ibuprofen is employed as the active ingredient in formulations containing this class of excipients, such formulations have advantageous storage properties over similar formulations comprising ibuprofen itself. This effect is surprising as pharmaceutically acceptable salts of ibuprofen would not be expected to exhibit superior storage characteristics compared to ibuprofen when combined with a carrier of this type.

Accordingly, the present invention provides a pharmaceutical formulation comprising a dispersion of a pharmaceutically acceptable salt of ibuprofen in a carrier which comprises a hydrophilic material selected from an alcohol or an ester thereof and a polyhydric alcohol or an ester thereof.

The pharmaceutically acceptable salt is suitably provided as a uniform dispersion in the hydrophilic material. Desirably the particles of the salt of ibuprofen are in intimate admixture with the hydrophilic material in a uniform mixture or blend. The improved stability properties are particularly shown by a reduction in side-products formed on storage. The side-products are caused by an interaction between the active ingredient and the hydrophilic material.

Preferably the hydrophilic material in accordance with the invention comprises non-ionic surfactants, melt-fill excipients, monohydric alcohols, glycols, polygylcols and glycerol, and esters thereof. Preferred examples of non-ionic surfactants are polyoxyalkylene derivatives, especially polyoxyethylene sorbitan esters, polyoxyethylene castor oil derivatives, polyoxyethylene alkyl ethers or polyoxyethylene-polyoxypropylene block copolymers. The melt-fill excipients are solid excipients which may be heated until molten, combined with active ingredient and allowed to cool to form a solid dosage presentation. Preferred melt-fill excipients are glyceride and/or glycide esters of fatty acids, in particular saturated $C_{8-22}$ fatty acids. Preferred examples of monohydric alcohols and esters thereof are derived from aliphatic alcohols having from 1 to 12 carbon atoms, in particular 1 to 4 carbon atoms, especially ethanol and isopropyl palmitate. Preferred examples of glycols and esters thereof are derived from glycols having 2 to 12 carbon atoms, in particular 2 to 6 carbon atoms, especially triethylene glycol and propylene glycol. A preferred polyglycol is polyethylene glycol. Preferred examples of glycerides are glyceryl monostearate and mono-and/or di-and/or tri-glycerides of $C_{8-22}$ fatty acids. If desired, two or more hydrophilic excipients may be combined to provide a mixture.

Advantageous results have been obtained when the hydrophilic material comprises ethanol, triethylene glycol, propylene glycol, polyethylene glycol isopropyl palmitate, glycol monostearate, triglycerides of $C_{8-22}$ fatty acids, polyoxyethylated derivatives, especially polyoxyethylene sorbitan esters, polyoxyethylene castor oil derivatives or polyoxyethylene-polyoxypropylene block polymers. Particularly valuable storage properties are obtained when using ethanol, triethylene glycol, polyethylene glycol, polyoxyethylene-polyoxypropylene block copolymers or polyoxyethylated castor oil derivatives as the hydrophilic material. The most preferred formulations comprise polyethylene glycol, triglycerides of $C_{8-22}$ fatty acids, polyoxyethylene castor oil derivatives or polyoxyethylene-polyoxypropylene block copolymers.

Advantageous storage properties are observed by the use of the salt of ibuprofen when the hydrophilic material comprises a small proportion of the carrier. However, the effect is observed to a greater extent when the hydrophilic material provides significant proportion of the carrier, for example greater than 10%, ie. in the range 10-100% by weight. Preferably the hydrophilic material provides 50-100% by weight of the carrier, more preferably 70-100% by weight. Particularly advantageous storage properties are observed when the hydrophilic material provides 80-100% by weight of the carrier. In especially preferred compounds the carrier consists essentially of the hydrophilic material. Preferably the hydrophilic material provides 25-75% by weight of the formulation.

Suitably the ration of the pharmaceutically acceptable salt of ibuprofen to the hydrophilic material is in the range 1:20 to 20:1 parts by weight, especially 1:5 to 5:1 parts by weight. Particularly preferred formulations comprise the active ingredient in a ratio to the hydrophilic material of 1:3 to 1.5:1 parts by weight. A formulation according to the invention may suitably comprise 5-95% by weight of a pharmaceutically acceptable salt of ibuprofen and 95-5% by weight carrier. Preferably the formulations comprise 5-95% by weight of a salt of ibuprofen and 95-5% by weight hydrophilic material, more preferably 10-70% by weight of a salt of ibuprofen and 90-30% by weight hydrophilic material and especially 10 to 50% by weight of a salt of ibuprofen and 90-50% by weight hydrophilic material.

Such formulations are particularly applicable to suppositories, topical formulations, non-aqueous liquid preparations including syrups, sprays and injectable suspensions and to oral presentations such as melt fill solid dosage forms and soft or hard gelatin capsules, which may contain solid, semi-solid or liquid preparations.

Optionally, other pharmaceutically acceptable excipients may also be incorporated into the carrier in a formulation according to the invention. Such pharmaceutically acceptable excipients may be added to facilitate the manufacture of suitable dosage forms of the formulation and/or modify the rate of drug dissolution. A skilled person will appreciate the excipients and the proportions thereof necessary in the formulation. For example, where a formulation according to the invention is desired to be in the form of a gel, liquid or semi-solid presentation, e.g. a cream or paste, the pharmaceutically acceptable formulation excipients may further include one or more of the following: water, wetting and emulsifying agents, suspending and thickening agents, humectants preservatives, flavourings and colouring agents. Where a formulation according to the invention is desired to be presented in solid form, in particular a liquid filled gelatin capsule or a suppository, the pharmaceutically acceptable excipients may include binders, thickeners, soluble and insoluble diluents, lubricants, flow aids and other oils fats and waxes as desired. Also suitable pharmaceutically acceptable excipients which produce accelerated rates of dispersion and dissolution may be added to a formulation according to the invention.

The formulations according to the invention may be prepared in unit dosage form. Such dosage units suitably contain the therapeutic equivalent of the pharmaceutically acceptable salt of ibuprofen as provided by 50-1200 mg, particularly 200-800 mg of ibuprofen.

Formulations according to the invention have activity as analgesics, anti-inflammatory and antipyretic agents.

Formulations according to the invention may be prepared by a variety of processes depending on the desired form of presentation. Any of the known processes for the production of solid or liquid presentations is applicable, for example the pharmaceutically acceptable salt of ibuprofen may be dispersed, optionally with other pharmaceutically acceptable excipients in a liquid or in a molten carrier which is then allowed to cool, or may be mixed with other formulation aids and compressed into a solid form.

The invention is illustrated by the following non-limitative examples.

In the Examples the hydrophilic materials are provided by materials having the following trade names:-

a) Witepsol E85 and Witespol H15 (glycerol esters of mixtures of saturated vegatable fatty acids in which lauric acid predominates) available from Dynamit Nobel, Slough, Berkshire;

b) Gelucire 35/10 and Gelucire 44/14 (partial glycerides and polyglycides of natural vegetable oil fatty acids) available from Gattefosse Co., Saint-Priest, France;

c) Tween 80 (polyoxyethylene sorbitan esters) available from Atlas Chemicals, Carshalton, Surrey;

d) Cremophor EL and Cremophor RH40 (polyoxyethylene castor oil derivatives) available from Blagden Campbell Chemicals, Croyden, Surrey;

e) Imwitor 742 (monoglycerides of saturated fatty acids from coconut oil [mainly octanoic and decanoic acids]) available from Dynamit Nobel, Slough, Berkshire;

f) Suppocire B (mono-/di-/triglycerides of vegetable oils and polyoxyethylene glycerides) available from Gattefosse Co., Saint-Priest, France;

g) Miglyol (triglycerides of medium-chain saturated fatty acids including linoleic acid (available from Dynamit Nobel, Slough, Berkshire;

h) Jelot 64 (glycerol polyoxyethylene glycol palmito stearate) available from Gattefosse Co., Saint-Priest, France;

i) Pluronic L62 (polyoxyethylene-polyoxypropylene block copolymer) available from Blagden Campbell Chemicals Ltd, Croydon, Surrey; and

j) Labrafil M2130CS ($C_{12-18}$ ethoxylated saturated glycerides) available from Gattefosse Co., Saint Priest, France.

Example 1

A formulation containing the following ingredients:-

|  | % w/w |
|---|---|
| Ibuprofen (as the sodium salt) | 20 |
| Glycerol esters (Witepsol E85) | 45 |
| Glycerol esters (Witepsol H15) | 35 |

was prepared by combining the glycerol esters excipients to form a homogeneous material. The sodium salt of ibuprofen was then blended with the carrier to form a uniform mixture.

Example 2

A formulation containing the following ingredients:-

|  | % w/w |
|---|---|
| Ibuprofen (as the sodium salt) | 50 |
| Polyethylene glycol (M.wt = 300) | 50 |

was prepared by blending the polyethylene glycol with the sodium salt of ibuprofen to form a uniform mixture. The mixture was filled by pipette into gelatin capsules.

Example 3

A formulation containing the following ingredients:-

|  | % w/w |
|---|---|
| Ibuprofen (as the sodium salt) | 54.4 |
| Partial glycerides and polyglycides (Gelucire 44/14) | 40.6 |
| Polyoxyethylene sorbitan esters (Tween 80) | 5 |

was prepared by warming the partial glycerides and polyglycides excipient and sorbitan esters excipient to 60°C. The sodium salt of ibuprofen was added with stirring to form a homogeneous molten mixture. The mixture was filled by pipette into gelatin capsules.

In the same way may be prepared capsules containing ibuprofen (sodium salt) [52% w/w], partial glycerides and polyglycides (Gelucire 44/14) [38% w/w] and polyoxyethylene castor oil derivatives (Cremophor EL) [10% w/w] and also capsules containing ibuprofen (sodium salt) [55% w/w], partial glycerides and polyglycides (Gelucire 44/14) [40% w/w] and stearyl alcohol [5% w/w].

Example 4

A formulation containing the following ingredients:-

|  | % w/w |
|---|---|
| Ibuprofen (as the sodium salt) | 64 |
| Monoglycerides (Imwitor 742) | 32 |
| Glycerol | 4 |

was prepared by intimately blending the sodium salt of ibuprofen, the monoglycerides excipient and glycerol before filling into gelatin capsules.

Example 5

A formulation containing the following ingredients:-

|  | % w/w |
|---|---|
| Ibuprofen (as the sodium salt) | 32 |
| Glyceryl monostearate | 5 |
| Glycerol esters (Suppocire B) | 63 |

was prepared by warming the glycerol esters excipient and glyceryl monostearate to 50°C and adding the sodium salt of ibuprofen with stirring to form a homogeneous molten mixture. The mixture was filled into suppository cavities and allowed to cool.

Example 6

A formulation containing the following ingredients:-

|  | % w/w |
|---|---|
| Ibuprofen (as the sodium salt) | 30 |
| Polyethylene glycol (m.wt = 1000) | 15 |
| Polyethylene glycol (m.wt = 4000) | 50 |
| Triglyceride (Miglyol) | 5 |

was prepared by warming the polyethylene glycol excipients to 60°C and adding the triglyceride excipient component and the sodium salt of ibuprofen with stirring to form a homogeneous molten mixture. The mixture was filled into suppository cavities.

Example 7

A formulation containing the following ingredients:-

|  | % w/w |
|---|---|
| Water | 46.9 |
| Ibuprofen (as the sodium salt) | 15.6 |
| Glycerol stearate (Jelot 64) | 11.7 |
| Stearyl alcohol | 6.2 |
| Glycerol esters (Labrafil M2130CS) | 11.7 |
| Sorbitol BP | 2.3 |
| Colloidal silicon dioxide (Aerosil 200) | 3.9 |
| Phenoxylethanol | 1.6 |
| Butylated hydroxytoluene | 0.003 |

was prepared by heating the glycerol esters excipient to 60°C and adding the butylated hydroxytoluene, stearyl alcohol and sodium salt of ibuprofen with stirring. The remaining ingredients were added to the water and stirred until dissolved or dispersed. The aqueous dispersion was added to the glycerol esters mixture and cooled whilst stirring.

Comparative Examples

Studies were carried out to investigate the stability in various carriers of ibuprofen as the sodium salt compared with ibuprofen.

Example A

A comparison was made between formulations containing ibuprofen and formulations containing ibuprofen as the sodium salt with each of the carriers listed in Table 1 below. The formulations contained ibuprofen or ibuprofen as the sodium salt in an amount in the range 10-25% w/w, the carrier providing the remainder of the formulation (90-75% w/w).

6

Table 1

| Carrier | Trade Name |
|---|---|
| Polyethylene glycol (molecular weight 300) | |
| Propylene glycol | |
| Ethanol | |
| Isopropyl palmitate | |
| Polyoxyethylene sorbitan esters | Tween 80 |
| Polyoxyethylene castor oil derivatives | Cremophor EL<br>Cremophor RH40 |
| Monoglycerides | Imwitor 742 |
| Polyoxyethylene-polyoxypropylene block copolymer | Pluronic L62 |

The samples were stored at temperatures in the range 20-40°C for five weeks. The samples were then examined by thin layer chromatography carried out on precoated silica gel plates, eluted with trichloroethane: methyl acetate; glacial acetic acid (8:1:1), and visualised at 254 nm in a UV cabinet to determine whether the samples contained side-products. Traces of side-products are an indication of instability.

With each of the carriers listed in Table 1 above, the formulation comprising the sodium salt of ibuprofen showed a decreased number of side-products thus indicating increased stability compared to the formulation comprising ibuprofen.

Example B

Additional comparative tests were carried out between formulations comprising 25% w/w ibuprofen or ibuprofen as the sodium salt and 75% w/w carrier.

The carriers employed are listed in Table 2 below.

7

TABLE 2

| Carrier | Trade Name |
|---|---|
| Polyethylene glycol (molecular weight 6000) | |
| Stearyl alcohol | |
| Glycerol | |
| Partial glyceride and polyglycide esters of natural vegetable oil fatty acids | Gelucire 44/14 |
| Triglycerides | Miglyol 812 |

The samples were maintained at temperatures in the range 50-70°C for ten days and examined by the same thin layer chromatography method as described in Example A to determine whether the samples contained traces of side-products.

With each of the carriers listed in Table 2 above, the formulation comprising the sodium salt of ibuprofen showed a decreased level of side-products thus indicating improved stability over formulations comprising ibuprofen.

Example C

The following comparative tests showed the formulation containing ibuprofen as the sodium salt to contain fewer side-products than the formulation containing ibuprofen, using the carriers listed in Table 3 below. The presence of additional side-products was determined by the thin layer chromatography was determined in Example A. The samples were stored at a temperature in the range 5-40°C for 12 months.

The formulations contained 10-25% w/w ibuprofen or ibuprofen as the sodium salt and 90-75% w/w carrier.

8

## TABLE 3

|                                                                      | Trade Name                        |
|----------------------------------------------------------------------|-----------------------------------|
| Polyoxyethylene-polyoxypropylene block copolymer                     | Pluronic L62                      |
| Triethylene glycol                                                   |                                   |
| Glyceryl monostearate                                                |                                   |
| Stearyl alcohol                                                      |                                   |
| Polyethylene glycol (molecular weight 300)                           |                                   |
| Monoglycerides of satuarated fatty acids from coconut oil            | Imwitor 742                       |
| Partial glycerides and polycides of natural vegetable oil fatty acids | (Gelucire 35/10 (Gelucire 44/14  |
| Polyoxyethylene castor oil derivatives                               | (Cremophor RH40 (Cremophor EL    |
| Propylene glycol                                                     |                                   |
| Isopropyl palmitate                                                  |                                   |
| Ethanol                                                              |                                   |
| Triglycerides of medium-chain saturated fatty acids including linoeic acid | Miglyol 812                |
| Polyoxyethylene sorbitan esters                                      | Tween 80                          |

9

**Claims**

1. A pharmaceutical formulation comprising a dispersion of a pharmaceutically acceptable salt of ibuprofen in a carrier which comprises a hydrophilic material selected from an alcohol or an ester thereof and a polyhydric alcohol or an ester thereof.

2. A formulation according to claim 1 wherein the hydrophilic material is selected from a polyoxyalkylene derivative, a monohydric alcohol or an ester thereof, a gylcol or an ester thereof, a polyglycol or an ester thereof, or glycerol or an ester thereof.

3. A formulation according to either one of claims 1 and 2 wherein the hydrophilic material provides at least 50% of the carrier.

4. A formulation according to either one of claims 1 and 2 wherein the carrier consists essentially of the hydrophilic material.

5. A formulation according to any one of claims 1 to 4 wherein the hydrophilc material is selected from polyoxyethylene sorbitan esters, polyoxyethylene castor oil derivatives, polyoxyethylene-polyoxypropylene block copolymers, partial glyceride and/or polyglycide esters of $C_{8-22}$ fatty acids, ethanol, ispropyl palmitate, triethylene glycol, propylene glycol, polyethylene glycol, glyceryl monostearate, monoglycerides of $C_{8-22}$ fatty acids, diglycerides of $C_{8-22}$ fatty acids and triglycerides of $C_{8-22}$ fatty acids.

6. A formulation according to any one of claims 1 to 5 wherein the hydrophilic material is selected from ethanol, triethylene glycol, propylene glycol, polyethylene glycol, polyoxyethylene sorbitan esters, isopropyl palmitate, glyceryl monostearate, triglycerides of $C_{8-22}$ fatty acids, polyoxyethylene castor oil derivatives and polyoxyethylene-polyoxypropylene block copolymers.

7. A formulation according to any one of claims 1 to 6 wherein the ratio of the pharmaceutically acceptable salt of ibuprofen to the hydrophilic material is in the range 1:5 to 5:1 parts by weight.

8. A formulation according to any one of claims 1 to 7 wherein the pharmaceutically acceptable salt is the sodium salt of ibuprofen.

9. A formulation according to any one of claims 1 to 8 presented as an oral or topical dosage form.

10. The use of a pharmaceutically acceptable salt of ibuprofen in an ibuprofen formulation comprising a carrier which comprises a hydrophilic material selected from an alcohol or an ester thereof and a polyhydric alcohol or an ester thereof to stabilise the formulation.

## CLAIMS FOR THE FOLLOWING CONTRACTING STATES GR, ES GREECE & SPAIN

1. A process to prepare a pharmaceutical formulation comprising a dispersion of a pharmaceutically acceptable salt of ibuprofen in a carrier which comprises a hydrophilic material selected from an alcohol or an ester thereof and a polyhydric alcohol or an ester thereof, comprising dispersing the salt of ibuprofen in the hydrophilic material to produce a uniform mixture, and forming the mixture into a unit dosage form.

2. A process according to claim 1 wherein the hydrophilic material is selected from a polyoxyalkylene derivative, a monohydric alcohol or an ester thereof, a glycol or an ester thereof, a polyglycol or an ester thereof, and glycerol or an ester thereof.

3. A process according to either one of claims 1 and 2 wherein the hydrophilic material provides at least 50% of the carrier.

4. A process according to either one of claims 1 and 2 wherein the carrier consists essentially of the hydrophilic material.

5. A process according to any one claims of 1 to 4 wherein the hydrophilic material is selected from polyoxyethylene sorbitan esters, polyoxyethylene castor oil derivatives, polyoxyethylene-polyoxypropylene block copolymers, partial glyceride and/or polyglycide esters of $C_{8-22}$ fatty acids, ethanol, ispropyl palmitate, triethylene glycol, propylene glycol, polyethylene glycol, glyceryl monostearate, monoglycerides of $C_{8-22}$ fatty acids, diglycerides of $C_{8-22}$ fatty acids and triglycerides of $C_{8-22}$ fatty acids.

6. A process according to any one of claims 1 to 5 wherein the hydrophilic material is selected from ethanol, triethylene glycol, propylene glycol, polyethylene glycol, polyoxyethylene sorbital esters, ispropyl palmitate, glyceryl monostearate, triglycerides of $C_{8-22}$ fatty acids, polyoxyethylene castor oil derivatives and polyoxyethylene-polyoxypropylene block copolymers.

7. A process according to any one of claims 1 to 6 wherein the ratio of the pharmaceutically acceptable salt of ibuprofen to the hydrophilic material is in the range 1:5 to 5:1 parts by weight.

8. A process according to any one of claims 1 to 7 wherein the pharmaceutically acceptable salt is the sodium salt of ibuprofen.

9. A process according to any one of claims 1 to 8 wherein the unit dose is presented as an oral or topical dosage form.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 206 291 (KLINGE PHARMA GmbH) * Page 3, lines 5-15; claims 1-4 * | 1-6,9, 10 | A 61 K 31/19 A 61 K 47/00 |
| X | DE-A-3 340 347 (THE UPJOHN CO.) * Page 4, lines 11-23; page 5, lines 20-24; page 7, line 22 - page 8, line 6; example 12 * | 1,2,5,6 ,8-10 | |
| P,X | EP-A-0 250 802 (MERCKLE GmbH) * Column 1, line 35 - column 2, line 29; examples 1,2 * | 1-9 | |
| Y | EP-A-0 178 436 (DOLORGIET GmbH & CO.KG) * Claims 1-4 * | 1-10 | |
| P,Y | EP-A-0 215 423 (DOLORGIET GmbH & CO.KG) * Column 3, line 39 - column 4, line 22; claims 1-10 * | 1-10 | |
| P,Y | EP-A-0 223 369 (THE BOOTS CO.) * Page 10, lines 3-13; example 12 * | 1-10 | TECHNICAL FIELDS SEARCHED (Int. Cl.4) A 61 K |

The present search report has been drawn up for all claims

| Place of search | Date of completion of the search | Examiner |
|---|---|---|
| THE HAGUE | 19-05-1988 | TZSCHOPPE,D.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)